(19) Europäisches Patentamt
European Patent Office
Office européen des brevets

(11) **EP 3 421 988 A1**

(12) **EUROPEAN PATENT APPLICATION**

(43) Date of publication:
**02.01.2019 Bulletin 2019/01**

(51) Int Cl.:
***G01N 33/00*** *(2006.01)*

(21) Application number: **17178822.7**

(22) Date of filing: **29.06.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Domesens srl**
**20900 Monza (MB) (IT)**

(72) Inventors:
• GUARINO, Daniele
  **20851 Lissone (MB) (IT)**
• GOBBO, Sebastiano
  **20900 Monza (MB) (IT)**

(74) Representative: **Spadaro, Marco et al**
**Cantaluppi & Partners S.r.l.**
**Via Strobel, 8**
**20133 Milano (IT)**

(54) **DEVICE AND A METHOD FOR THE CONTINUOUS MEASUREMENT OF ONE OR MORE TREE STATICITY**

(57)     The present invention describes a device and a method for continuously measuring the staticity of one or more trees. The device according to the invention comprises in essence a triple axis movement sensor, a data processing unit, a data transmission unit, and a data receiving unit, wherein the components are operatively connected. An anemometer may be operatively connected to the data receiving unit.

Fig. 1

## Description

The present invention relates to the agricultural sector, particularly arboriculture, and more particularly to a device and to a method for continuously measuring the staticity of one or more trees.

## Background to the invention

Trees in an urban environment do not have ideal conditions for optimal plant growth because of considerable interference from humans and suboptimal environmental conditions. Soil compaction, the impermeability of paved surfaces, atmospheric pollution, vandalism, inadequate training of the workers looking after general and special maintenance of green spaces, poor quality of original materials from the nursery and glaring mistakes committed when planting are just some of the causes of degradation of the plant component of urban areas.

To preserve a town's green heritage status, suitable operations need to be put in place for maintaining or renovating the trees they contain, and the state of health of several specimens should also be monitored. Monitoring the structural stability of the trees starts with taking a census of the whole estate, and this is even more essential in urban centres, not only out of a natural interest in their health but also and especially in order to guarantee the safety of people and to prevent damage to property. The investigation method currently most used in Europe and the USA is VTA (Visual Tree Assessment), as developed by Mattheck (Mattheck & Broeler, 1994). This is based on an initial visual diagnosis of the general condition of every single plant, followed - if deemed necessary by the worker - by using equipment to assess the extent of the internal changes. The method is based on the fact that internal static defects in a tree are related to specific, externally visible symptoms. Visual analysis, i.e. the identification and coding of these symptoms, is the trickiest part of the analysis. This first phase makes it possible to identify specimens that require further diagnosis using equipment, to confirm and quantify the structural defects that most affect the stability of the tree. This method requires highly experienced workers and is in any case affected by subjectivity.

The ultimate aim of instrumental analysis is to check and quantify the extent of damage or defects that might compromise the tree's stability and, based on those results and considerations of the tree's physiological conditions and any pathological signs, to assess its susceptibility to structural failure.

Various instruments are currently available on the market that are suitable for aiding diagnosis of degradative processes that cause the wood to decay. The instruments are based on various principles: variations in the velocity of shockwaves or ultrasound in the wood (sonic and ultrasonic pulse hammers, sonic tomography), thermal infrared emission in the woody tissues (thermography), variations in the electrical conductivity of the wood (Shigometer), the wood's resistance to penetration by a probe (penetrometer), sampling of the wood and destructive tests with portable dynamometers (fractometer).

At present, the instruments most commonly used for investigating the internal state of the wood are penetrometers, also known as wood densitometers and sonic tomographs.

The use of wood densitometers is based on the concept that the resistance of wood to perforation depends on its density, and therefore greater penetrability is observed in degraded wood than in healthy wood. The undisputed advantage of such an instrument is the objectivity of the information, although interpreting the results requires expert personnel. The drawback relates chiefly to the finding of point-specific, circumscribed information, requiring repeated analyses that are therefore considerably invasive.

Electronic hammers, based on measuring the speed at which a shockwave passes through a section, also have similar limitations to penetrometers as regards their point-specific, invasive nature. Moreover, even though this is a practical, fast method, it is approximate and not objective, because the manual hammer blows may not be uniform.

A partial improvement over the use of an electronic tap hammer can be achieved with sonic tomography. In this case, the investigation is no longer point-specific but covers the whole section of the trunk; however, it has drawbacks similar to those of a hammer' on account of its invasiveness and the non-uniformity of the manually set wave frequency, and it is certainly less applicable in practice than wood densitometer probes.

A more accurate assessment of the mechanical conditions of the wood can be obtained by using a fractometer, which measures the bending moment and the bending angle of a core of wood extracted using a Pressler auger. The obvious limitation of this methodology is the invasiveness of coring (portions of wood with diameters of over 0.5 cm), together with the poor reliability of responses, which are too dependent on the humidity level and the growth stage of the tree.

Some instruments are based on variations in the electrical properties of the wood, particularly on the different impedance of damaged wood compared with healthy wood. These include the Shigometer and the Vitamat. In both cases, test responses are very reliable but the need to pierce the trunk at several points in order to insert the electrodes makes them highly invasive; furthermore, calibrating the instruments is a lengthy and costly task, since the electrical properties of the wood depend on the species, the moisture content of the tree, its physiological conditions and growth stage, and the internal and environmental temperature.

Thermography is based on the concept that abnormal wood tissues have different degrees of thermal conductivity with respect to sound ones. As in the previous case, the major limitation of this methodology is the variability of responses depending on factors that are intrinsic and extrinsic to the specimen in question.

In radar, a transmitting and a receiving antenna, which are rotated about the trunk, detect any delays in transmission of the waves caused by cavities, and X-ray and gamma-ray emissions are based on the same concept. Both methods are very reliable and non-invasive, but their use in an urban environment is limited to experimental applications in view of the high costs and legislative constraints imposed in relation to the use of electromagnetic and radioactive waves.

The limitations of the various techniques mentioned above result, to varying degrees, from the point-specific nature of the analysis, relatively marked invasiveness, unreliable repeatability and the need for operator experience in interpreting the results.

The limitation common to all the instruments is that they cannot easily make diagnoses of the plant's hypogeal organs: the root system is difficult to inspect and complete degradation of one or more structural roots can frequently result in the sudden uprooting of the specimen, without any warning signs in terms of either growth or structure.

To this end, recent years have seen the introduction, both in urban environments and in general for ornamental trees, of the use of the controlled pulling test as a procedure for assessing the stability of trees in order to determine as accurately as possible the potential for uprooting of the root ball or breakage of a tree trunk.

The most commonly used methods were formulated by Lothar Wessolly (Wessolly, L. - M. Erb 1998. Handbuch der Baumstatik und Baumkontrolle. Patzer Verlag, Berlin, Germany) and known by the abbreviations SIA (Statics Integrated Assessment) and SIM (Statics Integrated Method); they were developed using a complex modelling approach borrowed from engineering.

The controlled pulling test consists in subjecting the tree to semi-static traction stress using a block and tackle connected to a fixed anchor point, and measuring the relationship between the force exerted and the stress induced in the tree.

The value obtained measures - or rather - estimates the maximum load that the tree can bear before breaking or being uprooted, and at the same time calculations can be made to indicate the critical wind speed that might cause that uprooting.

The stresses studied are the inclination of the root ball, measured by positioning inclinometers close to the root collar, and elongation of the fibres, measured by means of strain gauges positioned at a certain height on the outer surface of the trunk. In order not to damage the tree's mechanical strength, the stress exerted on the specimen is contained within certain predetermined limits, but is nevertheless sufficient to determine the critical breaking or uprooting load of the tree from the stress-strain ratio measured.

The strength of the wind on the tree ($F_{wind}$) is calculated by the formula generally used for estimating the wind strength on an object:

$$F_{wind} = f \cdot c_w \cdot \rho/2 \cdot \sum \left( u_z{}^2 \cdot A_{h_z} \right)$$

- $f$ = turbulence factor
- $c_w$ = aerodynamic coefficient
- $u_z$ = wind speed
- $A_{h_z}$ = surface area of the crown exposed to wind at the height $h_z$ above the ground

By preparing a modelling analysis, the method makes it possible to estimate the maximum critical speed at which the tree might break or overturn.

In practice, some of these values, for example the surface area of greatest exposure of the crown or the height of the centre of gravity of the crown, are not easy to determine precisely; furthermore, the air density, which requires point-specific measurements, and other values such as the aerodynamic coefficient or the turbulence factor, are substantially difficult to determine and therefore liable to excessively great approximation, which may modify the final result.

These coefficients are therefore estimated on the basis of experience (seeking to maintain the safest possible conditions) and therefore the results deduced from applying them cannot be considered absolutely precise, but rather, as a reference magnitude known as the "safety factor", for the likely ability of the tree to withstand a certain wind speed.

As regards the value of the safety factor (and therefore also the value of the critical velocity from which said safety factor is deduced) obtained from the controlled pulling test, we should remember that the values used relate to the mechanical characteristics of the wood of a certain species of tree, deduced from specific publications, and we should therefore note that the relationship between the modulus of elasticity and the tensile stress is not completely unequivocal, but can vary within a certain range from tree to tree, even of the same species, according to the environmental factors that influence the tree's development, such as pedoclimatic conditions, the rooting site and various other factors.

All the instruments used to date for assessing the stability of trees have some limitations, which in some cases are decisive in terms of the reliability of the measurement made, which is sometimes reduced to an estimate suffering from a wide margin of error and poor predictability.

In the case of instruments that measure the extent of degradation of the inner wood, either with various types of penetrometer or by tomography using electrical or sound waves, even when the measurements are reliable, the operator's technical training in choosing test points and in interpreting and assessing the data is crucial for making a judgement as to the tree's stability.

In the case of the controlled pulling test, however meticulously the procedure is performed, the margins of uncertainty of the end result are fairly high, owing to a series of factors such as the subjective or forced choice of the anchor point, the pulling direction and, just as importantly, the impossibility of measuring the effect of turbulence created by the context and the highly variable calculation procedures, which depend on the use of "standard" data that cannot be adapted to the context.

Hence, the need arises to identify an instrument and a methodology allowing measurement, rather than approximate assessment, of the tree's response to a given stress (wind, snow), without applying a simulated load or using parametric formulas comprising highly subjective factors.

All the measurements that can be made relate to a point in time and to a given moment, whilst when managing critical states arising from the trees' stability, it is essential to use constant monitoring that will detect "in real time" even tiny variations in static configuration in order to intervene when necessary to reduce the danger.

CN103903400 describes a system for monitoring the inclination of a tree at high risk of falling and warning of dangerous situations, on reaching a predetermined alarm value. The system comprises a three-axis MEMS accelerometer that sends the data acquired via the GSM network.

Far too often in our towns, following weather events - never so violent as in recent years - a large number of trees are broken or brought down, causing great damage to property and even harming people, and other trees do not fall but are removed because they have visible damage; however, any trees that have suffered significant damage without revealing it externally escape inspection, and are highly dangerous specimens since even a mild weather event occurring later could result in them unexpectedly collapsing.

Therefore, there is still a perceived need to overcome the technical problems seen above, and in particular to provide an instrument for measuring the staticity of the tree or of several trees, which is non-invasive, does not require complex calibration and/or highly specialised personnel, and gives direct measurements without having to consult other sources.

In particular, there is a perceived need for an instrument that can take measurements even when unattended and, above all, continuously, allowing even remote monitoring in real time and for several periods of time, so as to build up a "history" of the tree being monitored and, if necessary, notifying of any static critical states with sufficient advance warning.

**Summary of the invention**

The present invention solves the above-mentioned technical problems by means of an apparatus and a system as described below.

The following definitions are provided in the context of the present invention.

"Apparatus": means a component comprising one or more devices cooperating to perform a more complex function, for example, a monitoring component and a computer. An apparatus can also be integrated into a system.

"System": means an assembly of one or more apparatuses and/or devices that cooperate to perform a more complex function.

"Operatively connected" means a connection that is able to transmit a data flow between one or more input and/or output ports. The connection can be of any suitable type, wired or wireless.

"Signal processing device": means any device capable of processing input signals and producing output signals. This device can be a processor, incorporated into a more complex apparatus or system, for example a computer.

The apparatus and the system forming the subject matter of the present invention are as defined in the independent claims. The dependent claims refer to various embodiments of the invention.

The present invention also relates to a system comprising a series of pieces of apparatus as defined above, which are operatively connected to form a monitoring network.

The present invention also relates to a method for continuously measuring the movement of a tree, using the above-mentioned apparatus.

The present invention also relates to a method for continuously measuring the movement of a group of trees, using the above-mentioned system.

One subject matter of the present invention is an apparatus, also referred to below as a TSS (tree staticity sensor), comprising the following devices:

    a) a triple axis movement sensor;

    b) a data processing unit;

    c) a data transmission unit;

    d) a data receiving unit;

wherein components a) to d) are operatively connected.

In one embodiment of the invention, said apparatus further comprises:

    e) an anemometer;

wherein component e) is operatively connected to component d).

In one embodiment of the invention, in said apparatus, said data transmission unit c) operates over a public band, preferably an ultra-narrow band (UNB), for example 868 MHz.

In one embodiment of the invention, said apparatus comprises a power supply unit, for example a battery or a photovoltaic, piezoelectric, triboelectric or thermoelectric source, preferably a photovoltaic film.

In one embodiment of the invention, said apparatus comprises a tracking unit.

Another subject matter of the present invention is a monitoring network comprising a series of pieces of apparatus that are described above and are operatively connected.

Another subject matter of the present invention is a method for continuously measuring the staticity of a tree or a group of trees, comprising the following steps:

a) continuously measuring the movement of a tree or a group of trees, said measurement being determined as acceleration (m/s$^2$);

b) optionally continuously measuring the wind speed (m/s);
wherein said steps a) and b) are carried out at the same time T; leading to

c) measuring the amplitude and direction of the movements of the tree or a group of trees.

In one embodiment of the present invention, said method further comprises

before stage a):

a$^0$) measuring the initial position of the tree or a group of trees;

and after stage c):

d) measuring the difference between the position of the tree or a group of trees in step a$^0$) and the position of the tree or a group of trees in step c).

In one embodiment of the present invention, said method further comprises the generation of an alarm signal if the difference measured in step d) is not 0 or is greater than a specific critical value for the tree species measured.

Another subject matter of the present invention is a method for continuously measuring the staticity of a tree or a group of trees using the apparatus described above, comprising the following steps of:

a) positioning the sensor a) on said tree or said group of trees;

b) entering data into the data processing unit b) that relates to the positioning height of said sensor, the cardinal point, data relating to the dimensions of the tree or said group of trees and the botanical species;

c) resetting the sensor a) when the tree or said group of trees is static;

d) continuously acquiring acceleration data in said unit b) and converting it into degrees of deviation from the initial reset point for an assessment period;

e) processing the average movement data in said unit b), which average is obtained during the assessment period, to eliminate momentary point events and avoid false alarms;

f) sending the movement data recorded during the assessment period to the unit d), via the unit c).

In one embodiment of the present invention, said method further comprises the following steps of:

g) sending a first-level alarm signal if the critical movement threshold defined on the basis of the characteristics of the tree or a group of trees is exceeded; and optionally

h) sending a second-level alarm signal if the values remain above the critical threshold; and optionally

i) analysing the movement data and comparing it with any exogenous data;

j) defining rules of behaviour of the tree or of said group of trees and monitoring, over time, any changes from the initial reset point;

k) dynamically calculating the tree's safety factor and sending a third-level alarm signal if the admissible tolerance limits are not observed.

Another subject matter of the present invention is the use of the apparatus or the network of apparatuses described above for implementing the above-mentioned methods.

These and other subjects of the present invention are now presented in detail in the following description, and by means of figures and examples.

**Description of the figures**

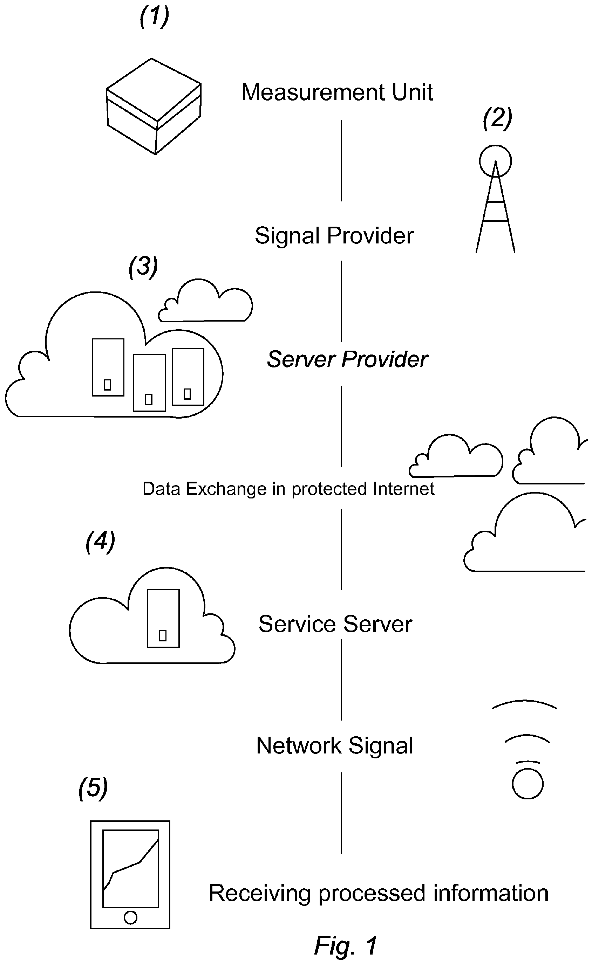

Fig. 1 shows an embodiment of the apparatus and system of the present invention.

**Detailed description of the invention**

The apparatus of the present invention essentially comprises the following devices:

a) a triple axis movement sensor (accelerometer);

b) a data processing unit;

c) a data transmission unit;

d) a data receiving unit;

components a) to d) being operatively connected.

The triple axis movement sensor is a device known as an accelerometer. In the present invention, this device is also called a staticity sensor.

This device is applied to the trunk of the tree whose movement, particularly inclination, is to be measured.

The device is fixed to the tree trunk by any suitable means.

The accelerometer is normally commercially available and the one considered by the skilled person to be most suitable can be used.

The staticity sensor TSS preferably has an independent power source so that the power supply can be maintained for an indefinite period. Various power sources can be provided, for example photovoltaic, piezoelectric, triboelectric or thermoelectric sources.

In particular, photovoltaic devices are of greatest interest since they are able to provide the greatest amount of energy having regard to the features of the subject matter.

The preferred types of available photovoltaic devices are plastics photovoltaic films, printed by printing techniques, for example as marketed by Ribes Tech Srl. In fact, in structural terms these are absolutely identical to a plastics film and are flexible and light. Their deposition techniques ensure the greatest freedom of shape and colour of the device, making it possible to take full advantage of the surface area available for recovering energy, the shape of the device being adapted to the shape and curvature of the surface. Finally, these technologies offer interesting advantages in diffused and low-intensity light conditions, such as below the foliage of a tree, allowing power generation even in conditions of limited and indirect sunlight.

However, if wished, the device can also be supplied with power from an external source.

In one embodiment of the invention, the TSS device can house a tracking system, for example a radio system based on Bluetooth Low Energy communication (iBeacon or eBeacon systems). This allows the TSS to communicate its proximity to a device capable of reading the signal from the TSS (for example a mobile telephone or a laptop computer). The closeness of an operator to a given tree is therefore checked and the operator can download all the information relating to that individual plant via a data connection.

In another embodiment of the invention, the apparatus described here essentially comprises the following devices:

a) a triple axis movement sensor (accelerometer);

b) a data processing unit;

c) a data transmission unit;

d) a data receiving unit;

e) an anemometer;

wherein components a) to d) are operatively connected and component e) is operatively connected to component d).

The anemometer device is also of a conventional, commercially available type.

The data processing unit is typically a processor. The processor can be incorporated into one of the devices making up the apparatus of the present invention, for example the accelerometer, which in this case becomes a data acquisition and processing unit.

The data transmission unit operates over any suitable support. Transmission can be over a telephone network (GSM), or over a public band, also defined as an ISM (industrial, scientific and medical) radio band, governed by the international convention of the ITU (International Telecommunication Union), to which the skilled person will refer. For example, in Europe the band is 868-870 MHz. In one embodiment of the invention, transmission over the public band uses the Sigfox protocol or the LoRa protocol. In another embodiment of the invention, the NarrowBand IoT (NB-IoT), as well as the recent 5G, can be used.

Sigfox uses a proprietary technology that allows communication using the ISM (industrial, scientific and medical) radio band on the 868 MHz frequency in Europe and on the 902 MHz frequency in the United States of America. This uses a wide-reaching signal that passes freely through solid objects, known as "ultra-narrowband", and requires little power; it is also called a "low-power wide-area network" (LPWAN). The network is based on "one-hop star" topology and requires a mobile operator to support the traffic generated. The signal can also be used to cover wide areas and to reach underground objects.

The ultra-narrowband (UNB) network is an energy-efficient, wide-range, narrowband network based on small transmitter devices and a dedicated antenna base station and it records the tree's movement when it is subjected to wind stress or burdened by the weight of snow, at intervals defined on a case-by-case basis, preferably by measuring at upwards of a second.

Advantageously, the apparatus according to the present invention can have a graphical interface that displays the tree movements recorded.

Another subject matter of the present invention is a method for continuously measuring the staticity of a tree, comprising the following steps:

a) continuously measuring the movement of a tree or a group of trees, said measurement being defined as acceleration (m/s$^2$);
b) optionally continuously measuring the wind speed (m/s);

said steps a) and b) being carried out at the same time T; leading to

c) measuring the amplitude and direction of movement of the tree or a group of trees.

According to the present invention, the principle of monitoring is that the measurements should be continuous, because if sampled at specific points, i.e. at preset moments, there is a risk that events and conditions occurring at times other than at the recording moment are not recorded, with the risk that a critical condition is considered normal because it was not recorded at that time. The device and the method of the present invention make the measurements at time intervals that can be considered "continuous", for example at intervals of at least one second.

The data acquired in steps a) and b) are processed by statistical methods so that they can be compared with historical data and with the zero staticity point defined when the TSS is installed, and they output the amplitude (in tenths of a degree) and the direction of the movements of the tree subjected to that given stress.

The amplitude of travel is calculated by interpolation of the various measurements in order to reduce the effect of noise, and is done by measuring the size of the Euler angles (roll-pitch-yaw) in degrees.

This mode produces real and continuous measurements of the response of that particular tree - even when there is degeneration of the internal tissues that could reduce the resistant base portion - to specific external stresses, including any turbulence generated by the context in which the tree takes root (practically impossible to measure).

The data thus obtained allow the most correct use, for the purposes of prediction, of the formulas for identifying the value of the critical stress as given in the literature, since they are stripped of highly subjective factors (air density, foliage roughness coefficient, fluid resistance, frictional resistance and form drag etc.), which by nature lead to very variable and consequently unreliable results. The critical value is detected by the monitoring system, i.e. by the central server where the data are processed, where the correlation tables will be implemented.

The information provided by the TSS via a graphical interface available to the operator is crucial for constant monitoring of woodlands; it reveals whether or not the tree returned to its initial position of inertia after having undergone varying degrees of stress to which it was subjected.

At the time of installation, TSS records the position of the tree (moment of inertia), defined here as point "zero". With reference to that point, the instrument measures the deviations caused by the movement of the tree under stress.

At the end of the stressing phase the tree must, as a resilient structure, return to its initial position. Otherwise, if there has been serious damage to the root system, the tree cannot return to its initial position and this factor is immediately notified by the TSS with an alarm.

Therefore, in one embodiment of the invention, the method according to the present invention also provides the further steps:

before step a):

$a^0$) measuring the initial position of the tree or a group of trees;

and after step c):

d) measuring the difference between the position of the tree or a group of trees in step $a^0$) and the position of the tree or a group of trees in step c).

In another embodiment of the invention, the method described above also provides the further steps:

and after step d):

generating an alarm signal if the difference measured in step d) is not 0 or is greater than a specific critical value for the tree species measured.

The measurements recorded continuously by the accelerometer are processed using a calculation algorithm by which they can be converted into real values of the tree's displacement from its inertia point and compared with the critical alert threshold defined according to the tree's characteristics entered in the software and, in the case of interface with exogenous data, allows abnormal behaviour of the tree to be assessed.

The calculation procedure is described below.

1. Entering the positioning height of the TSS, the cardinal point, data relating to tree dimensions and botanical species;
2. resetting the apparatus when the tree is static;
3. continuously acquiring acceleration data and converting it into degrees of deviation from the initial reset point for an assessment period;
4. processing the average movement data obtained during the assessment period (ideally from 5 to 10 minutes), to eliminate momentary point events and avoid false alarms;
5. sending the movement data recorded during the assessment period;
6. sending a first-level alarm signal if the critical movement threshold defined on the basis of the characteristics of the tree is exceeded;
7. sending a second-level alarm signal if values remain above the critical threshold;
8. analysing the movement data and comparing it with any exogenous data (anemometric and pluviometric data, safety factor calculated from previous analytical tests);

9. defining rules of behaviour of the tree and monitoring over time any changes from the initial reset point (i.e. deriving from an analysis of the individual reactions of the tree in real, variable stress conditions from factors inherent in the specimen, such as morphology, wood characteristics, aerodynamics, etc., or from the rooting context, which may differ from that expected from using estimation systems based on standard data from the literature or subjective adjustments to the various parameters required for calculating the wind stress);

10. dynamically calculating the tree's safety factor and sending a third-level alarm signal if the admissible tolerance limits are not observed.

The safety factor must be sufficient for the stress conditions detected, particularly weather conditions, which can be extremely calm or stormy. Hence the dynamic adjustment to the safety factor, which could vary according to the stresses suffered and/or the altered conditions of the tree.

With this method, by alerting the operator of any variation in the position of the tree, the TSS performs a very important function of implementing a constant monitoring programme, as currently required by many public authorities.

In one embodiment, the present invention provides a method for continuously measuring the staticity of a tree or a group of trees by using the apparatus or system described above; said method comprising the following steps:

a) positioning the sensor a) on said tree or said group of trees;

b) entering data into the data processing unit b) that relates to the positioning height of said sensor, the cardinal point, data relating to the dimensions of the tree or said group of trees and the botanical species;

c) resetting the sensor a) when the tree or said group of trees is static;

d) continuously acquiring acceleration data in said unit b) and converting it into degrees of deviation from the initial reset point for an assessment period;

e) processing the average movement data obtained during the assessment period in said unit b) to eliminate momentary point events and avoid false alarms;

f) sending the movement data recorded during the assessment period to the unit d), via the unit c).

In another embodiment of the invention, the method described here comprises the further steps of:

g) sending a first-level alarm signal if the critical movement threshold defined on the basis of the characteristics of the tree or a group of trees is exceeded; and optionally

h) sending a second-level alarm signal if the values remain above the critical threshold; and optionally

i) analysing the movement data and comparing it with any exogenous data;

j) defining rules of behaviour of the tree or of said group of trees and monitoring, over time, any changes from the initial reset point;

k) dynamically calculating the tree's safety factor and sending a third-level alarm signal if the admissible tolerance limits are not observed.

The fields of application of the TSS are:

- Public and private arboreal asset management: in relation to the crucial contribution made to reducing danger posed by the instability of trees, increasing safety of exploitation and monitoring valuable tree specimens in a highly critical condition.

- Professional sphere: the TSS apparatus is ideal for incorporating the instrument currently available to the professional for carrying out tree stability tests, applying the VTA method or other methods.

- Insurance issues: the TSS apparatus performs the function of constantly monitoring the stability conditions of tree specimens, notifying of the slightest variations in static configuration, to allow timely intervention in order to reduce danger.

- Applied research in arboriculture: the TSS apparatus is an effective support for gathering study data in order to understand numerous phenomena connected with how the tree reacts to very different stresses and how said tree reacts in an urban environment, so as to select the most suitable species and rooting substrates.

The apparatus according to the present invention belongs to the category of IoT (internet of things) systems.

Therefore, the apparatus described here lends itself to the construction of a system comprising several pieces of apparatus connected via the internet.

This system is useful for monitoring a group of trees, for example in parks, gardens and forests.

All the systems are connected to the monitoring system either directly (peer-to-peer), sending data directly to the server of the communication system, or via a gateway that acts as a concentrator. This condition depends on the data communication system chosen: Sigfox uses a network provided by a specialised operator (NetTrotter in Italy) that allows each individual device to communicate directly with the server, as can those operating with the GSM/GPRS services of TelCo, whereas if LoRa is used, the data converge at a gateway that handles communication with the server. All options are available, depending on the communication system chosen, which is a component of the system.

With reference to Fig. 1, a detailed description will now be given of one embodiment of the present invention.

The apparatus (1) comprising the accelerometer is installed on a tree (not shown in the figure) and takes the measurements of tree movement. The data transmission unit is connected to the public network (2) and sends the data to a server of the network provider (3); the data are recovered by the user's server (4), where data processing takes place. Data exchange between the device and the service server and data recovery by the user's server from the service server are preferably all done in encrypted and protected mode. The user's server, which can also be hosted in the cloud, sends the data to the display device (5), for example a computer, a handheld device or a smart phone via a wired or wireless system.

To improve calculation of the safety factor, in one embodiment of the invention the anemometer e) (not shown in the figure) is suitably positioned relative to the tree, or trees where several trees are being measured, and is operatively connected to the data receiving unit d).

In one embodiment, the components of the apparatus of the present invention, all commercially available, can have the following features, it being understood that other components having the same function can also be used.

- Processor

Dimensions and weight

- 41 x25x9mm
- Native Arduino Processor

Arduino-compatible

- ATmega328P processor @ 8 MHz
- Pre-loaded Arduino bootloader
- Arduino IDE support

- Housing and support for solderless components

- 7x GPIO, I$^2$C/SPI, 2x 10-bit ADC, 3x PWM
- 5VUSB/500mA, 3.3V/200mA power output
- Up to 18x GPIOs, 6x 10-bit analogue inputs or 6x PWMs

- RGB LED

- RGB LED, up to 2240 mcd, 120° viewing angle

- Wireless

- "XBee" form factor, for housing similar devices
- Wireless support: Bluetooth Low Energy 4.0, WiFi, Bluetooth 2.1, XBee, GSM/GPRS, Sigfox, LoRa
- 2-channel Over-the-Air Programming (OTAP)
- 5-second programming via XBee, Bluetooth, WiFi

- Ultra Low Power Management System

- 155mAh rechargeable lithium-ion battery
- 1 A switchable RF LDO
- 330 nA system deep sleep
- Autonomy in sleep mode: 2-3 years
- Autonomy in communication: 6 months (1 data/minute) Integrated battery charger
- Single-cell Li-ion USB charger
- Battery Over Current, Charge & Discharge protect
- Battery level indicator, charge status, USB monitor
- Charge time: 1.5 hours

Plastics film photovoltaic cell

- 2 x 6V photovoltaic cells

Beacon

- eBeacon/iBeacon circuit powered by photovoltaic cell
- Wireless on Bluetooth Low Energy 4.0

Three-axis accelerometer (features)

- Ultralow power: less than 23 $\mu$A during measurement and 0.1 $\mu$A in standby mode at VS = 2.5 V (typical);
- Automatically graduated consumption according to bandwidth;
- User-selectable resolution
- Fixed 10-bit resolution;
- Full resolution, where the resolution increases with "g" range, up to 13-bit resolution at $\pm$16 g (maintaining 4 mg/ LSB scale factor over the whole "g" range);
- Memory management system with FIFO technology, minimising the host processor load;
- Detection of single and double blows;
- Activity/inactivity monitoring;
- Detection of free-fall phenomenon;

- Power supply range: 2.0 V to 3.6 V
- I/O voltage range: 1.7 V to VS;
- SPI (3- and 4-wire) and I$^2$C digital interfaces;
- Flexible procedure for mapping interrupts to other pins;
- Measurement range selectable by serial commands;
- Bandwidth selectable by serial commands;
- Wide operating temperature range (-40°C to +85°C);
- Operating resistance to 10.000 g shock;
- Pb free/RoHS compliant;
- Small and thin: 3 mm x 5 mm x 1 mm LGA package.

The accelerometer is preferably small, thin and energy-efficient with high measurement resolution (13-bit) up to $\pm$ 16 g. The digital output is in 16-bit two's complement format and is accessible either via an SPI (3- or 4-wire) or an I$^2$C digital interface.

The accelerometer is suited to mobile device applications. It measures the static acceleration of gravity in tilt-sensing applications, as well as the dynamic acceleration resulting from movement or impacts. Because of its high resolution (3.9 mg / LSB), changes in inclination of less than 1.0° can be measured.

Various special detection functions are provided. The activity and inactivity sensors detect the presence or absence of movement by comparing acceleration on any axis with the sensitivity thresholds set by the user. The agitation sensor detects single or double blows in any direction. The free-fall sensor detects whether the device is falling.

These functions can be mapped individually to one of the two interrupt output pins. An integrated memory-management system with a 32-level first in, first out (FIFO) buffer can be used for storing the data, to reduce as far as possible the activity of the host processor and to decrease the overall energy consumption.

The energy-efficient modes permit power management based on intelligent movement with a detection threshold and measurement of active acceleration with extremely low power dissipation.

Transmission component

- SIGFOX Ready™;
- Frequency range = ISM 868 MHz;
- Sensitivity in receiving mode =-126 dBm;
- Modulation: (G)FSK, 4(G)FSK, GMSK. OOK;
- Max output power: +14 dBm;
- Energy-efficient when radio is active: 13/16 mA RX; 37 mA TX @ +10 dBm;
- Power supply = 2.3 to 3.3 V;
- LGA25 (25.4 x 12.7 x 3.81 mm) Land Grid Array package.

These devices are low-current, high-performance Sigfox™ gateways. The combination of a powerful radio transceiver and an ARM Cortex M3 baseband processor achieves extremely high performance, while maintaining ultra-low current consumption when active or on standby. The device offers extraordinary RF sensitivity of 126 dBm, supplying exceptional output power up to +14 dBm with unparalleled TX efficiency. The versatility of the device provides the gateway function from a local narrowband ISM network up to the long-distance Sigfox™ ultra narrowband at no extra cost.

The wide range of analogue and digital interfaces available in the module allows any application to be easily interconnected to the Sigfox™ network. The energy-efficient LVTTL UART, the I$^2$C bus, the multiple timers with PWM pulse/functionality counting input, high-resolution/high-speed ADC and DAC, together with the numerous GPIO pins, are able to monitor any type of external activators and sensors.

Featuring an AES cryptography engine and a DMA controller, the powerful 32-bit ARM Cortex-M3 baseband processor is able to implement highly complex and secure protocols in an efficient environment and in an extremely energy-efficient and extremely low power-dissipation mode.

Photovoltaic plastics film

- plastics film;
- Nanotechnology inks for circuit printing;
- "Typographic" deposition printing process.

The plastics film is made photovoltaic through "photolito" printing techniques using special inks. The deposition techniques ensure the greatest freedom of shape and colour of the device and this makes it possible to make best use of the available surface area for energy recovery, the shape of the device being adapted to the shape and curvature of the surface area available. The film also offers appealing advantages under the diffused and low-intensity light conditions typical of artificially lit indoor environments, or direct or reflected sunlight.

Beacon

- BLE circuit - Bluetooth Low Energy programmed to send iBeacon (Apple)/ eBeacon (Android) messages.

The Beacon technology, based on BLE (Bluetooth Low Energy), allows beacon devices to transmit and receive short messages over short distances. The device continuously sends short identification messages (equivalent to the beacon function for navigation, hence the name) allowing a receiver (e.g. smartphone) to detect it via an app. The standard information sent by the beacons consists of a UUID, and just a major or minor value. For example, UUID: B9407F30-F5F8-466E-AFF9-25556B57FE6D Major ID: 1, Minor ID: 2, sent every fraction of a second, where UUID is a unique identifier of the device, and in our case identifies the tree being monitored.

A few examples of application of the present invention are given below.

Roadside trees

The apparatus is installed on roadside trees of a fair size, chosen as representative samples of the whole population along the roadside; after a period of recording and subsequently processing the data, the degree of stability is defined and if necessary maintenance operations are prepared accordingly. The monitoring period after the operation makes it possible to assess whether or not the proposed objective has been achieved.

Trees at risk of falling

The apparatus is installed on trees that, because of their general condition and stability, have been classified as "highly or extremely likely to fall" (classes C-D and D of the P.a.C. [propensity to fall] classification of the Società Italiana di Arboricoltura) for which felling is advised, but which have a high botanical, historical or sentimental value. In these cases, the application of the TSS defers felling and initiates a monitoring period to assess the tree's actual degree of resilience, with the possibility of intervening at the first sign of abnormality.

Trees of historical value

The apparatus is installed on sample trees in historic parks, where respect for the monumental nature of these specimens, including any morphological anomalies, requires minimally intensive maintenance methods and all efforts aimed at preserving these trees for as long as possible. In these cases the TSS is able to notify those responsible, in real time, when a tree has exceeded its structural limit and only in this case does intervention take place. This kind of approach would avoid any felling of trees dictated by emotional reactions or, worse still, incorrect assessments.

Trees in at-risk areas

The apparatus is installed on trees located in areas with a high risk of damage to people or property (town squares, schools, major road networks, etc.) and in this case too the TSS is able to notify those responsible, in real time, when a tree has exceeded its structural limit, activating the planned procedures in these cases.

Intense weather events

The apparatus is installed on trees planted in areas that have suffered from the passage of a particularly intense weather event, in order to check the actual static resilience of these surviving specimens.

Special installations

The apparatus is installed on trees for experimental purposes to assess species of trees whose dimensions and cultivation methods are best suited to a certain type of rooting substrate, for example in the case of hanging gardens or special sites in an urban environment, in order to guarantee a tree structure having "normal" resilience, and not having to do without trees or resort to elaborate anchorage systems.

**Claims**

1. Apparatus for measuring the staticity of one or more trees, comprising the following devices:

   a. a triple axis movement sensor;
   b. a data processing unit;
   c. a data transmission unit;
   d. a data receiving unit;

   wherein components a) to d) are operatively connected.

2. Apparatus according to claim 1, further comprising:

   e) an anemometer;

   wherein component e) is operatively connected to component d).

3. Apparatus according to either claim 1 or claim 2, wherein said data transmission unit c) operates over a public band.

4. Apparatus according to claim 3, wherein said public band is an ultra-narrow band (UNB).

5. Apparatus according to claim 4, wherein said ultra-narrow band is 868 MHz.

6. Apparatus according to any one of claims 1 to 5, comprising a power supply unit.

7. Apparatus according to claim 6, wherein said power supply unit is selected from the group consisting of a battery and a photovoltaic, piezoelectric, triboelectric or thermoelectric source.

8. Apparatus according to claim 7, wherein said power supply unit is a photovoltaic film.

9. Apparatus according to any one of claims 1 to 8, comprising a tracking unit.

10. Monitoring network comprising a series of pieces of apparatus that are defined in any one of claims 1-9 and are operatively connected.

11. Method for continuously measuring the staticity of a tree or a group of trees, comprising the following steps:

    a. continuously measuring the movement of a tree or a group of trees, said measurement being defined as acceleration ($m/s^2$);
    b. optionally continuously measuring the wind speed (m/s);

wherein said steps a) and b) are carried out at the same time T; leading to

    c. measuring the amplitude and direction of the movements of the tree or a group of trees.

12. Method according to claim 11, further comprising:

    before step a):

        $a^0$) measuring the initial position of the tree or a group of trees;

    and after step c):

        d) measuring the difference between the position of the tree or a group of trees in step $a^0$) and the position of the tree or a group of trees in step c).

13. Method according to either claim 11 or claim 12, further comprising the generation of an alarm signal if the difference in step d) is not 0 or is greater than a specific critical value for the tree species measured.

14. Method for continuously measuring the staticity of a tree or a group of trees using the apparatus of any one of claims 1 to 9, comprising the following steps:

    a. positioning the sensor a) on said tree or said group of trees;
    b. entering data into the data processing unit b) that relates to the positioning height of said sensor, the cardinal point, data relating to the dimensions of the tree or said group of trees and the botanical species;
    c. resetting the sensor a) when the tree or said group of trees is static;
    d. continuously acquiring acceleration data in said unit b) and converting it into degrees of deviation from the initial reset point for an assessment period;
    e. processing the average movement data in said unit b), which average is obtained during the assessment period, to eliminate momentary point events and avoid false alarms;
    f. sending the movement data recorded during the assessment period to the unit d), via the unit c).

15. Method according to claim 15, comprising the further steps of:

    g) sending a first-level alarm signal if the critical movement threshold defined on the basis of the characteristics of the tree or a group of trees is exceeded; and optionally
    h) sending a second-level alarm signal if the values remain above the critical threshold; and optionally
    i) analysing the movement data and comparing it with any exogenous data;
    j) defining rules of behaviour of the tree or of said group of trees and monitoring, over time, any changes from the initial reset point;
    k) dynamically calculating the tree's safety factor and sending a third-level alarm signal if the admissible tolerance limits are not observed.

*(1)*

Measurement Unit

*(2)*

Signal Provider

*(3)*

*Server Provider*

Data Exchange in protected Internet

*(4)*

Service Server

Network Signal

*(5)*

Receiving processed information

*Fig. 1*

Europäisches
Patentamt

European
Patent Office

Office européen
des brevets

# EUROPEAN SEARCH REPORT

Application Number

EP 17 17 8822

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | CN 103 903 400 A (CHENGDU MICRO ENVIRON ENVIRONMENTAL MONITORING EQUIPMENT CO LTD) 2 July 2014 (2014-07-02) | 1,3-15 | INV. G01N33/00 |
| Y | * abstract * <br> * figure 1 * | 2 | |
| Y | ALPO HASSINEN ET AL: "A prism-based system for monitoring the swaying of trees under wind loading", AGRICULTURAL AND FOREST METEOROLOGY., vol. 90, no. 3, April 1998 (1998-04), pages 187-194, XP055417206, NL ISSN: 0168-1923, DOI: 10.1016/S0168-1923(98)00052-5 | 2 | |
| A | * page 188, right-hand column, paragraph 2 - page 189, left-hand column, paragraph 2 * <br> * figure 1 * | 1,3-15 | |
| A | Britni Michelle ET AL: "An Estimation of Critical Wind Speed and a Calculation of Mechanical Properties for Two Australian Eucalypts A thesis submitted in fulfilment of the requirements for the degree of Masters of Engineering by Research (Mechanical & Manufacturing)", , October 2016 (2016-10), XP055417221, Retrieved from the Internet: URL:http://researchbank.rmit.edu.au/eserv/rmit:161842/Green.pdf * page 46 * * page 57 - page 58 * * figures 22,37 * | 1-15 | TECHNICAL FIELDS SEARCHED (IPC) <br><br> G01N |

-/--

The present search report has been drawn up for all claims

| Place of search | Date of completion of the search | Examiner |
|---|---|---|
| The Hague | 23 October 2017 | Baranski, Jörg |

EPO FORM 1503 03.82 (P04C01)

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KEN JAMES ET AL: "Measuring tilt of tree structural root zones under static and wind loading", AGRICULTURAL AND FOREST METEOROLOGY., vol. 168, 2013, pages 160-167, XP055417213, NL ISSN: 0168-1923, DOI: 10.1016/j.agrformet.2012.09.009 * page 161, left-hand column, paragraph 7 - right-hand column, paragraph 3 * ----- | 1-15 | |
| A | US 8 184 015 B2 (LILIEN JEAN-LOUIS [BE]; DESTINE JACQUES [BE]; UNIV LIEGE [BE]) 22 May 2012 (2012-05-22) * column 5, line 54 - column 6, line 12 * ----- | 1-15 | |
| A | US 2014/338447 A1 (SHARPE JOHNATHAN CHARLES [NZ] ET AL) 20 November 2014 (2014-11-20) * paragraph [0071] * * paragraph [0089] * * figures * ----- | 1-15 | TECHNICAL FIELDS SEARCHED (IPC) |

The present search report has been drawn up for all claims

| Place of search | Date of completion of the search | Examiner |
|---|---|---|
| The Hague | 23 October 2017 | Baranski, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 17 8822

This annex lists the patent family members relating to the patent documents cited in the above-mentioned European search report.
The members are as contained in the European Patent Office EDP file on
The European Patent Office is in no way liable for these particulars which are merely given for the purpose of information.

23-10-2017

| Patent document cited in search report | | Publication date | Patent family member(s) | | Publication date |
|---|---|---|---|---|---|
| CN 103903400 | A | 02-07-2014 | NONE | | |
| US 8184015 | B2 | 22-05-2012 | CA | 2618505 A1 | 22-03-2007 |
| | | | DK | 1938159 T3 | 19-12-2016 |
| | | | EP | 1938159 A1 | 02-07-2008 |
| | | | ES | 2603839 T3 | 01-03-2017 |
| | | | NO | 339621 B1 | 16-01-2017 |
| | | | PL | 1938159 T3 | 28-04-2017 |
| | | | US | 2009243876 A1 | 01-10-2009 |
| | | | WO | 2007031435 A1 | 22-03-2007 |
| | | | ZA | 200801282 B | 28-01-2009 |
| US 2014338447 | A1 | 20-11-2014 | AU | 2014270036 A1 | 21-01-2016 |
| | | | CA | 2913171 A1 | 27-11-2014 |
| | | | EP | 2999407 A2 | 30-03-2016 |
| | | | US | 2014338447 A1 | 20-11-2014 |
| | | | WO | 2014188273 A2 | 27-11-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 103903400

**Non-patent literature cited in the description**

- **WESSOLLY, L. - M.** Handbuch der Baumstatik und Baumkontrolle. Patzer Verlag, 1998